(19) Europäisches Patentamt
European Patent Office
Office européen des brevets

(11) Veröffentlichungsnummer: **0 030 287**
**A1**

(12) # EUROPÄISCHE PATENTANMELDUNG

(21) Anmeldenummer: **80107100.2**

(22) Anmeldetag: **15.11.80**

(51) Int. Cl.³: **C 07 C 143/78**, C 07 C 127/15, C 07 C 125/067, C 07 F 9/24, C 07 C 103/46, C 07 F 9/28, A 01 N 53/00

(30) Priorität: **29.11.79 DE 2948024**

(43) Veröffentlichungstag der Anmeldung: **17.06.81 Patentblatt 81/24**

(84) Benannte Vertragsstaaten: **BE CH DE FR GB IT LI NL**

(71) Anmelder: **BAYER AG, Zentralbereich Patente, Marken und Lizenzen, D-5090 Leverkusen 1, Bayerwerk (DE)**

(72) Erfinder: **Gallenkamp, Bernd, Dr., Claudiusweg 5, D-5600 Wuppertal 1 (DE)**
Erfinder: **Schröder, Rolf, Dr., Pahlkestrasse 17, D-5600 Wuppertal 1 (DE)**
Erfinder: **Lürssen, Klaus, Dr., August-Kierspel-Strasse 89, D-5060 Bergisch-Gladbach 2 (DE)**

(54) **1-Amino-cyclopropancarbonsäure-Derivate, Verfahren zu ihrer Herstellung, ihre Verwendung als Pflanzenwachstumsregulatoren und solche Derivate enthaltende Mittel.**

(57) 1-Amino-cyclopropancarbonsäure-Derivate der Formel

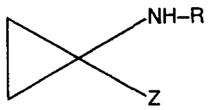

(I)

in welcher
Z und R die in der Beschreibung angegebenen Bedeutungen haben, mehrere Verfahren zur Herstellung der Stoffe der Formel (I) sowie deren Verwendung zur Regulierung des Pflanzenwachstums.

EP 0 030 287 A1

BAYER AKTIENGESELLSCHAFT 5090 Leverkusen, Bayerwerk

Zentralbereich
Patente, Marken und Lizenzen Dü-by-c

Ib

**BEZEICHNUNG GEÄNDERT**
**siehe Titelseite**

1-Amino-cyclopropancarbonsäure-Derivate, Verfahren zu ihrer Herstellung und ihre Verwendung als Pflanzenwachstumsregulatoren

Die vorliegende Erfindung betrifft neue 1-Amino-cyclopropancarbonsäure-Derivate, mehrere Verfahren zu ihrer Herstellung sowie ihre Verwendung zur Regulierung des Pflanzenwachstums.

Es ist bereits bekannt geworden, daß bestimmte N-Acylmethionin-derivate das Wachstum von Pflanzen beeinflussen (vgl. DE-OS 2 712 832). Die Wirkung dieser Verbindungen ist jedoch, insbesondere bei niedrigen Wirkstoffkonzentrationen und Aufwandmengen, nicht immer zufriedenstellend.

Es wurden nun neue 1-Amino-cyclopropancarbonsäure-Derivate der Formel

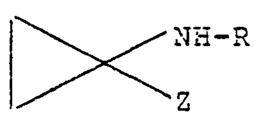

(I)

in welcher

Z für Cyano oder für den Rest $-CO-R^1$ steht, worin

$R^1$ für Hydroxy, Alkoxy, Alkenoxy, Alkinoxy, Aralkoxy, Aryloxy, oder für den Rest $-O^{\ominus}M^{\oplus}$ steht, wobei

$M^{\oplus}$ für ein Metallionenäquivalent oder für ein gegebenenfalls substituiertes Ammonium-, Sulfonium- oder Phosphonium-ion steht, und

$R^1$ weiterhin für den Rest $-N\begin{smallmatrix}R^2\\R^3\end{smallmatrix}$ steht, worin

$R^2$ und $R^3$ unabhängig voneinander für Wasserstoff oder für gegebenenfalls substituierte Reste aus der Reihe Alkyl, Cycloalkyl, Alkenyl, Alkinyl, Aralkyl oder Aryl stehen oder

$R^2$ und $R^3$ zusammen mit dem N-Atom an das sie gebunden sind, für einen gegebenenfalls substituierten, gesättigten oder ungesättigten, gegebenenfalls benzanellierten Mono- oder Bicyclus, welcher gegebenenfalls 1 bis 3 weitere Stickstoffatome oder ein Sauerstoff- oder Schwefelatom als Heteroatom(e) enthält, stehen und

R für gegebenenfalls substituiertes Alkyl, gegebenenfalls substituierten Aralkyl, einen gegebenenfalls

substituierten heterocyclischen Rest oder den Rest $-CO-R^4$ steht, worin

$R^4$ für substituiertes Alkyl, gegebenenfalls substituiertes Aryl, einen gegebenenfalls substituierten Aminorest, für gegebenenfalls substituiertes Alkoxy oder für gegebenenfalls substituiertes Aryloxy steht, und

R weiterhin für den Rest $-S(O)_n-R^5$ steht, worin

n für 0, 1 oder 2 steht und

$R^5$ für gegebenenfalls substituiertes Alkyl, gegebenenfalls substituiertes Aryl oder für einen gegebenenfalls substituierten Aminorest steht, und

R ferner für den Rest 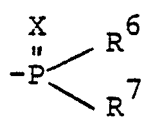 steht, worin

X für Sauerstoff oder Schwefel steht,

$R^6$ für gegebenenfalls substituiertes Alkyl, Aryl, Alkoxy oder Aryloxy steht und

$R^7$ für gegebenenfalls substituierte Reste aus der Reihe Alkoxy, Aryloxy, Alkylthio, Arylthio, Alkylamino, Arylamino, Alkyl oder Phenyl steht, und

R außerdem auch für Wasserstoff steht, mit der Maßgabe,

daß die Verbindung der Formel (I) dann salzartig mit einer Sauerstoffsäure von Schwefel oder Phosphor, welche gegebenenfalls organische Reste enthält, verbunden ist,

gefunden.

Weiterhin wurde gefunden, daß man 1-Amino-cyclopropancarbonsäure-Derivate der Formel (I) erhält, wenn man

a) 1-Amino-cyclopropancarbonsäure-Derivate der Formel

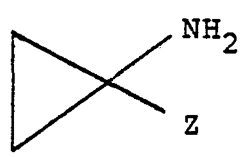

(II)

in welcher

Z die oben angegebene Bedeutung hat,

mit Verbindungen der Formel

$$Y-R^8 \qquad \text{(III)}$$

in welcher

$R^8$ die oben angegebene Bedeutung von R hat, jedoch nicht für Wasserstoff steht, und

Y für Halogen oder eine andere nucleofuge Gruppe steht,

gegebenenfalls in Gegenwart eines Säurebindemittels und gegebenenfalls in Gegenwart eines Verdünnungsmittels umsetzt, oder

b) 1-Amino-cyclopropancarbonsäure-chloride der Formel

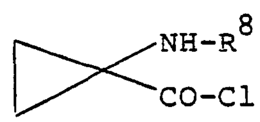

(IV)

in welcher

$R^8$ die oben angegebene Bedeutung von R hat, jedoch nicht für Wasserstoff steht,

mit Verbindungen der Formel

$$H-R^1 \quad (V)$$

in welcher

$R^1$ die oben angegebene Bedeutung hat,

gegebenenfalls in Gegenwart eines Säurebindemittels und gegebenenfalls in Gegenwart eines Verdünnungsmittels umsetzt, oder

c) 1-Amino-cyclopropancarbonsäure-Derivate der Formel

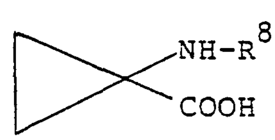

(VI)

in welcher

$R^8$ die oben angegebene Bedeutung von R hat, jedoch nicht für Wasserstoff steht,

mit Metallsalzen oder mit gegebenenfalls substituierten Ammonium-, Sulfonium- oder Phosphonium-Salzen gegebenenfalls in Gegenwart eines Säurebindemittels und gegebenenfalls in Gegenwart eines Verdünnungsmittels umsetzt, oder

d) 1-Amino-cyclopropancarbonsäure-Derivate der Formel

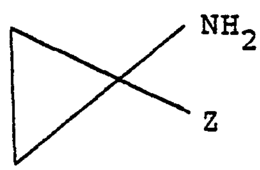

(II)

in welcher

Z die oben angegebene Bedeutung hat,

mit Sauerstoffsäuren von Schwefel oder Phosphor, welche gegebenenfalls organische Reste enthalten, gegebenenfalls in Gegenwart eines Verdünnungsmittels umsetzt.

Schließlich wurde gefunden, daß die neuen 1-Amino-cyclopropancarbonsäure-Derivate der Formel (I) sehr gut zur Regulierung des Pflanzenwachstums geeignet sind.

Überraschenderweise zeigen die erfindungsgemäßen 1-Amino-cyclopropancarbonsäure-Derivate der Formel (I) eine wesentlich bessere pflanzenwuchsregulierende Wirkung als aus dem Stand der Technik bekannte Verbindungen analoger Konstitution und gleicher Wirkungsrichtung.

Die erfindungsgemäßen 1-Amino-cyclopropancarbonsäure-Derivate sind durch die Formel (I) allgemein definiert. In dieser Formel steht Z für Cyano oder den Rest $-CO-R^1$. Der Rest $R^1$ steht vorzugsweise für Hydroxy, $C_1$-$C_{20}$-Alkoxy, $C_3$-$C_{10}$-Alkenoxy, $C_3$-$C_{10}$-Alkinoxy, Benzyloxy, Phenethoxy, Phenoxy oder für den Rest $-O^{\ominus}M^{\oplus}$, worin $M^{\oplus}$ vorzugsweise für ein Natrium- oder Kaliumion, ein Magnesium- oder Calcium-ionenäquivalent, für Ammonium, gegebenenfalls durch Halogen substituiertes Mono-, Di-, Tri- oder Tetraalkylammonium mit jeweils bis zu 4 Kohlenstoffatomen pro Alkylrest steht, wobei auch zwei Alkylreste zusammen mit dem N-Atom einen Ring bilden können, $R^1$ steht weiterhin für den Rest $-N{<}^{R^2}_{R^3}$, worin $R^2$ und $R^3$ unabhängig voneinander vorzugsweise für Wasserstoff, $C_1$-$C_6$-Alkyl, $C_3$-$C_7$-Cycloalkyl, $C_3$-$C_6$-Alkenyl, $C_3$-$C_6$-Alkinyl, Benzyl, Phenethyl oder für gegebenenfalls durch Halogen, $C_1$-$C_4$-Alkyl, Nitro, Trifluormethyl, Cyano und/oder $C_1$-$C_4$-Alkoxy substituiertes Phenyl stehen. $R^2$ und $R^3$ stehen ferner zusammen mit dem Stickstoffaton, an das sie gebunden sind, vorzugsweise für einen gegebenenfalls durch $C_1$-$C_4$-Alkyl substituierten, gesättigten oder ungesättigten und/oder benzannellierten Monocyclus oder Bicyclus mit bis zu 15 Kohlenstoffatomen, welcher gegebenenfalls ein Sauerstoffatom und gegebenenfalls weitere Stickstoffatome als Heteroatome enthält.

R steht in der Formel (I) vorzugsweise für Alkyl mit 1 bis 20 Kohlenstoffatomen, gegebenenfalls durch Alkyl mit 1 bis 4 Kohlenstoffatomen, Halogen und/oder Alkoxy mit 1 bis 4 Kohlenstoffatomen substituiertes Benzyl oder für einen gegebenenfalls durch Alkyl mit 1 bis 4 Kohlenstoffatomen und/oder Halogen substituierten gesättigten oder ungesättigten heterocyclischen Rest mit 5 bis 7 Ringgliedern und bis zu 3 Stickstoffatomen und/oder mit Sauerstoff oder Schwefel als Heteroatome. R steht weiterhin für der Rest $-CO-R^4$, worin $R^4$ vorzugsweise für $C_1$-$C_4$-Alkoxy-carbonyl-methyl, $C_1$-$C_4$-Alkyl-carbonyloxymethyl, Formyloxymethyl, Halogen-$C_1$-$C_4$-alkyl, $C_1$-$C_4$-Alkoxy, Phenoxy, Mono- oder Di-($C_1$-$C_4$-alkyl)-amino steht. R steht ferner für den Rest $-S(O)_n-R^5$, worin n vorzugsweise für 2 steht und $R^5$ vorzugsweise für $C_1$-$C_4$-Alkyl, gegebenenfalls durch Halogen oder Methyl substituiertes Phenyl oder für Di-($C_1$-$C_4$-alkyl)-amino steht. R steht außerdem für den Rest

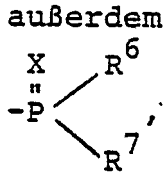

, worin X für Sauerstoff oder Schwefel steht, $R^6$ vorzugsweise für $C_1$-$C_4$-Alkyl oder $C_1$-$C_4$-Alkoxy steht und $R^7$ vorzugsweise für $C_1$-$C_4$-Alkyl, Phenyl, $C_1$-$C_4$-Alkoxy, $C_1$-$C_4$-Alkylthio oder $C_1$-$C_4$-Alkylamino steht. R steht schließlich auch für Wasserstoff, mit der Maßgabe, daß die Verbindung der Formel (I) salzartig mit vorzugsweise Schwefelsäure, einer gegebenenfalls halogen-substituierten $C_1$-$C_4$-Alkan-sulfinsäure oder -sulfonsäure, einer gegebenenfalls durch Halogen, Methyl und/oder Nitro substituierten Benzol- oder Naphthalin-sulfinsäure oder -sulfonsäure,

Phosphorsäure, einer gegebenenfalls halogen-substituierten $C_1$-$C_4$-Alkanphosphonsäure oder einer gegebenenfalls durch Halogen, Methyl und/oder Nitro substituierten Benzolphosphonsäure verbunden ist.

Verwendet man beispielsweise bei Verfahren (a) 1-Amino-cyclopropancarbonsäure-ethylester und Chloracetylchlorid, bei Verfahren (b) 1-Benzolsulfonylamino-cyclopropancarbonsäurechlorid und Dimethylamin, bei Verfahren (c) 1-Dimethylamino-carbonyl-amino-cyclopropancarbonsäure und N,N-Dimethyl-piperidiniumchlorid, sowie bei Verfahren (d) 1-Amino-cyclopropancarbonsäuremethylester und 2-Chlor-ethansulfinsäure als Ausgangsstoffe, so kann der Verlauf der Umsetzungen nach den erfindungsgemäßen Verfahren (a) bis (d) durch die folgenden Formelschemata wiedergegeben werden:

(a)

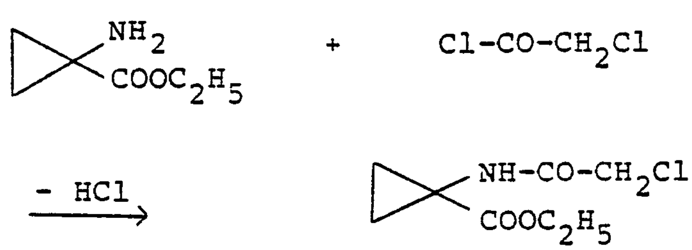


(b)

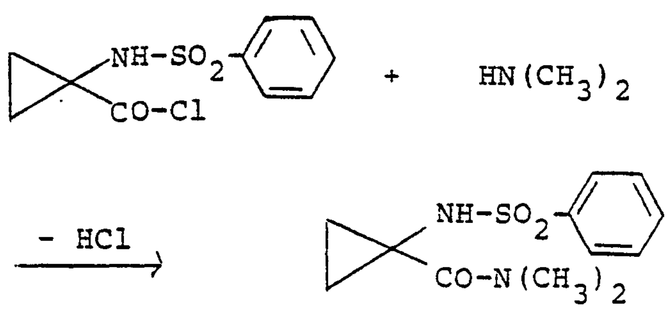

(c) 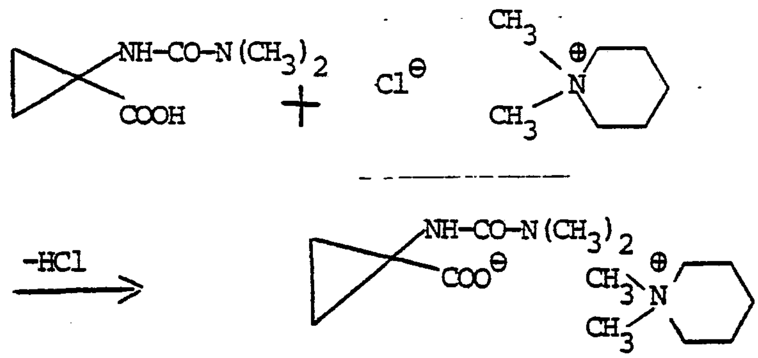


(d) 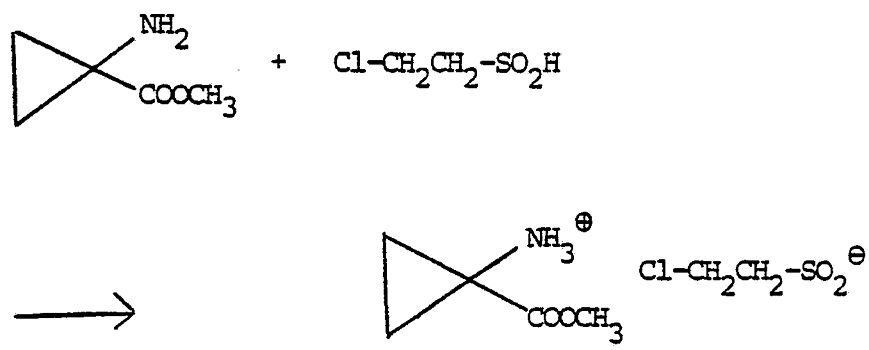

Die bei den Verfahren (a) bis (d) als Ausgangsstoffe zu verwendenden Verbindungen sind durch die Formeln (II) bis (VI) allgemein definiert. Z steht in der Formel (II) vorzugsweise für diejenigen Reste, die bereits im Zusammenhang mit der Beschreibung der erfindungsgemäßen 1-Amino-cyclopropancarbonsäure-Derivate vorzugsweise für Z genannt wurden. Der Rest $R^8$ steht in den Formeln (III), (IV) und (VI) vorzugsweise für diejenigen Bedeutungen von R, die bereits im Zusammenhang mit der Beschreibung der erfindungsgemäßen Stoffe vorzugsweise für R erwähnt wurden, jedoch steht $R^8$ nicht für Wasserstoff. Y steht in der Formel (III) vorzugsweise für Chlor, Brom oder $C_1$-$C_4$-Alkoxy. $R^1$ steht in der Formel (V) vorzugsweise für diejenigen Bedeutungen, die bereits im Zusammenhang mit der Beschreibung der erfindungsgemäßen Stoffe vorzugsweise für $R^1$ genannt wurden.

Die Verbindungen der Formel (II) sind bekannt oder lassen sich nach im Prinzip bekannten Verfahren, beispielsweise durch Hydrolyse von 1-Isocyano-cyclopropancarbonsäurederivaten herstellen (vgl. DE-OS 2 063 502 und Liebigs Ann. Chem. 1973, 611-618).

Als Beispiele für Verbindungen der Formel (II) seien genannt: 1-Amino-cyclopropancarbonsäure, deren Natrium-, Kalium-, Calcium-, Ammonium-, Dimethylammonium und Diethylammonium-salz, deren Methyl-, Ethyl-, Propyl-, Butyl- und Benzyl-ester sowie deren Amid, Dimethylamid und Diethylamid.

Die Verbindungen der Formel (III) sind bekannt. Als Beispiele seien genannt: Dimethylcarbaminsäurechlorid, Chlorameisensäuremethylester, -ethylester und -phenylester, Benzolsulfochlorid, p-Toluolsulfochlorid, O,O-Diethyl-phosphorsäurediesterchlorid, O-Ethylmethanphosphonsäureesterchlorid, O-Ethyl-N-isopropyl-thionophosphorsäureesteramidchlorid, Diethyl-thionophosphinsäurechlorid und O-Ethyl-S-propyl-thionophosphorsäurediesterchlorid, Chlor- und Brom-essigsäure-methyl und -ethylester, sowie Malonsäure-dimethylester und -diethylester.

Die bei Verfahren (b) als Ausgangsstoffe zu verwendenden 1-Amino-cyclopropancarbonsäurechloride der Formel (IV) sind noch nicht in der Literatur beschrieben. Man erhält

diese Verbindungen, indem man 1-Amino-cyclopropancarbonsäurederivate der Formel

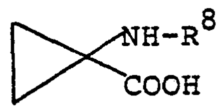

(VI)

in welcher

$R^8$ die oben angegebene Bedeutung hat,

mit einem Chlorierungsmittel, wie z.B. Thionylchlorid, gegebenenfalls in Gegenwart eines Verdünnungsmittels, wie z.B. Tetrachlorkohlenstoff und gegebenenfalls in Gegenwart eines Katalysators, wie z.B. Dimethylformamid oder Pyridin, bei Temperaturen zwischen 10 und 100°C umsetzt.

Die 1-Amino-cyclopropancarbonsäure-Derivate der Formel (VI) lassen sich nach dem erfindungsgemäßen Verfahren (a) herstellen.

Als Beispiele für die Ausgangsverbindungen der Formeln (IV) und (VI) seien genannt:
1-(Dimethylamino-carbonylamino)-, 1-(Methoxy-carbonylamino)-, 1-(Ethoxy-carbonylamino)-, 1-(Phenoxy-carbonylamino)-, 1-Benzolsulfonylamino-, 1-(p-Toluolsulfonylamino)-, 1-(O,O-Diethyl-phosphorylamino)-, 1-(O-Ethylmethanphosphonylamino)-, 1-(O-Ethyl-N-isopropylthionophosphorylamino)-, 1-Diethylphosphinylamino-, 1-(O-Ethyl-

S-propyl-thionophosphorylamino)-, 1-(Ethoxycarbonylmethyl-carbonylamino)-, 1-(Methoxycarbonylmethyl-carbonylamino)-, 1-(Methoxycarbonyl-methyl-amino)- und 1-(Ethoxycarbonyl-methyl-amino)-cyclopropancarbonsäure und -cyclopropancarbonsäurechlorid.

Die Ausgangsstoffe der Formel (V) sind bekannt.

Als Beispiele hierfür seien genannt: Methanol, Ethanol, n- und iso-Propanol, n-, iso-, sec- und tert.-Butanol, Allylalkohol, Propargylalkohol, Benzylalkohol, Dimethylamin, Diethylamin, Di-n-propylamin, Di-iso-propylamin, Di-n-butylamin, Di-isobutylamin, Methylethylamin, Methyl-n-propylamin, Methyl-isopropylamin, Methyl-n-butylamin, Methyl-isobutylamin, Methyl-sek.-butylamin, Ethyl-n-propylamin, Ethyl-isopropylamin, Ethyl-n-butylamin, Ethyl-iso-butylamin, Ethyl-sek.-butylamin, Propyl-butylamin, Propyl-isobutylamin, Propyl-sek.-butylamin, Allylamin, Diallylamin, Propargylamin, Dipropargylamin, N-Methyl-propargylamin, Dicyclopentylamin, Dicyclohexylamin, N-Methyl-cyclopentylamin, N-Methyl-cyclohexylamin, N-Ethyl-cyclopentylamin, N-Ethyl-cyclohexylamin, Dibenzylamin, N-Methyl-benzylamin, N-Ethyl-benzylamin, N-Propylbenzylamin, N-Butyl-benzylamin, N-Methyl-1-naphthylamin, N-Methyl-2-naphthylamin, N-Methyl-anilin, N-Ethyl-anilin, N-n-Propyl-anilin, N-iso-Propyl-anilin, N-n-Butyl-anilin, N-iso-Butyl-anilin, N-sek.-Butyl-anilin, N-Methyl-(2-methyl-phenyl)-amin, N-Methyl-(3-methyl-phenyl)-amin, N-Methyl-(4-methyl-phenyl)-amin, N-Methyl-(3-nitro-6-methyl-phenyl)-amin, N-Benzyl-anilin, Piperidin, 2-Methyl-,

3-Methyl-, 4-Methyl-, 2,4-Dimethyl-, 2,4,6-Trimethyl-, 2-Ethyl-, 4-Ethyl-, 2,4-Diethyl, 3,5-Dimethyl-, 2-Methyl-5-ethyl- und 2,4,6-Triethyl-piperidin, Pyrrolidin, 2-Methyl-pyrrolidin, 2,4-Dimethyl-pyrrolidin, 1,2,3,4-Tetrahydro-indolin, 2-Methyl-1,2,3,4-tetrahydroindolin, Perhydroindolin, 2-Methyl-perhydroindolin, 2,2-Dimethylperhydroindolin, 1,2,3,4-Tetrahydrochinolin, 2-Methyl-1,2,3,4-tetrahydrochinolin, Perhydrochinolin, 2-Methylperhydrochinolin, 1,2,3,4-Tetrahydro-iso-chinolin, Perhydroisochinolin, Morpholin, 3-Methyl-morpholin, 3,5-Dimethyl-morpholin, Perhydroazepin, 3,3,5-Trimethylperhydroazepin.

Als Beispiele für die bei Verfahren (c) einzusetzenden Salze seien genannt: Natrium-, Kalium-, Magnesium-, Calcium-, Ammonium-, Methylammonium-, Dimethylammonium-, Trimethylammonium-, Tetramethylammonium-, 2-Chlorethyltrimethylammonium-, N,N-Dimethyl-pyrrolidinium- und N,N-Dimethylpiperidinium-chlorid.

Als Beispiele für die bei Verfahren (d) einzusetzenden Sauerstoffsäuren von Schwefel oder Phosphor, welche gegebenenfalls organische Reste enthalten, seien genant: Schwefelsäure, Methansulfonsäure, 2-Chlor-ethansulfinsäure, Benzol- und Toluol-sulfonsäure, Naphthalin-1- und -2-sulfonsäure, Naphthalin-1,5-disulfonsäure, Phosphorsäure, Methanphosphonsäure, 2-Chlor-ethanphosphonsäure, Benzolphosphonsäure und 2-Nitro-benzolphosphonsäure.

Als Verdünnungsmittel kommen bei den erfindungsgemäßen Verfahren (a) bis (d) praktisch alle inerten organischen Lösungsmittel in Frage. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldienethylether, Tetrahydrofuran und Dioxan, Ketone wie Aceton, Methyl-ethyl-, Methyl-isopropyl- und Methylisobutyl-keton, Ester, wie Essigsäure-methylester und -ethylester, Nitrile, wie z.B. Acetonitril und Propionitril, Amide, wie z.B. Dimethylformamid, Dimethylacetamid und N-Methyl-pyrrolidon, sowie Dimethylsulfoxid, Tetramethylensulfon und Hexamethylphosphorsäuretriamid.

Es können gegebenenfalls auch Wasser und/oder Alkohole, wie z.B. Methanol, Ethanol, n- und iso-Propanol sowie n-, iso-, sec.- und tert.-Butanol als Verdünnungsmittel verwendet werden.

Als Säureakzeptoren können bei den erfindungsgemäßen Verfahren (a) bis (c) alle üblichen Säurebindemittel Verwendung finden. Besonders bewährt haben sich Alkalihydroxide, -carbonate und -alkoholate, wie Natrium- und Kaliumhydroxid, Natrium- und Kaliumcarbonat, Natrium- und Kaliummethylat bzw. -ethylat, ferner aliphatische, aromatische oder heterocyclische Amine, beispielsweise Triethylamin, Trimethylamin, Dimethylanilin, Dimethylbenzylamin und Pyridin.

Die Reaktionstemperatur kann bei den erfindungsgemäßen Verfahren (a) bis (d) innerhalb eines größeren Bereichs variiert werden. Im allgemeinen arbeitet man zwischen -20°C und 200°C, vorzugsweise zwischen 0°C und 150°C.

Die erfindungsgemäßen Verfahren werden im allgemeinen bei Normaldruck durchgeführt.

Bei Verfahren (a) setzt man auf 1 Mol 1-Amino-cyclopropancarbonsäure-derivat der Formel (II) 1 bis 3 Mol einer Verbindung der Formel (III) ein; bei Verfahren (b) werden je Mol 1-Amino-cyclopropancarbonsäurechlorid der Formel (IV) 1 bis 3 Mol einer Verbindung der Formel (V) eingesetzt; bei Verfahren (c) kommen auf 1 Mol 1-Amino-cyclopropancarbonsäure-derivat der Formel (VI) 0,3 bis 3 Mol Metall-, Ammonium-, Sulfonium- oder Phosphoniumsalze und bei Verfahren (d) auf 1 Mol 1-Amino-cyclopropancarbonsäure-derivat der Formel (II) ebenfalls 0,3 bis 3 Mol einer Sauerstoffsäure von Schwefel oder Phosphor.

Zur Durchführung der erfindungsgemäßen Verfahren (a) bis (d) werden die Reaktionskomponenten, gegebenenfalls in geeigneten Verdünnungsmitteln und gegebenenfalls unter Zugabe von Säurebindemittel, vermischt und die Reaktionsgemische werden bei den oben angegebenen Temperaturen bis zum Reaktionsende gerührt.

Die Aufarbeitung kann nach üblichen Methoden durchgeführt werden.

Zur Isolierung der freien Säuren der Formel (VI) - erhalten nach Verfahren (a) -, wird gegebenenfalls filtriert, die Lösung mit Mineralsäuren, wie z.B. Salzsäure, angesäuert und das in fester Form anfallende Produkt durch Absaugen isoliert.

Die Aufarbeitung der nach Verfahren (a) oder (b) erhaltenen Ester oder Amide der Formel (I) kann wie folgt durchgeführt werden:
Feste Produkte erhält man in reiner Form durch Abdestillieren des Lösungsmittels von der gegebenenfalls filtrierten Reaktionslösung und gegebenenfalls durch Umkristallisieren des Rückstandes.

Flüssige Produkte erhält man in reiner Form durch an die Umsetzung anschließende Destillation oder, indem man das Reaktionsgemisch gegebenenfalls mit Wasser und gegebenenfalls mit einem mit Wasser nicht mischbaren organischen Lösungsmittel verdünnt, nach dem Durchschütteln die organische Phase abtrennt, letztere mit Wasser wäscht, trocknet, filtriert, das Lösungsmittel unter vermindertem Druck sorgfältig abdestilliert und gegebenenfalls das zurückbleibende Produkt durch Vakuumdestillation reinigt.

Die nach den Verfahren (c) und (d) erhaltenen Produkte werden im allgemeinen durch Abdestillieren der Verdünnungsmittel isoliert.

Die erfindungsgemäß verwendbaren Wirkstoffe greifen in den Metabolismus der Pflanzen ein und können deshalb als Wachstumsregulatoren eingesetzt werden.

Für die Wirkungsweise von Pflanzenwachstumsregulatoren gilt nach der bisherigen Erfahrung, daß ein Wirkstoff auch mehrere verschiedenartige Wirkungen auf Pflanzen ausüben kann. Die Wirkungen der Stoffe hängen im wesentlichen ab von dem Zeitpunkt der Anwendung bezogen auf das Entwicklungsstadium der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in bestimmter gewünschter Weise beeinflussen.

Pflanzenwuchsregulierende Stoffe können zum Beispiel zur Hemmung des vegetativen Wachstums der Pflanzen eingesetzt werden. Eine derartige Wuchshemmung ist unter anderem bei Gräsern von wirtschaftlichem Interesse, denn dadurch kann die Häufigkeit der Grasschnitte in Ziergärten, Park- und Sportanlagen, an Straßenrändern, auf Flughäfen oder in Obstanlagen reduziert werden. Von Bedeutung ist auch die Hemmung des Wuchses von krautigen und holzigen Pflanzen an Straßenrändern und in der Nähe von Pipelines oder Überlandleitungen oder ganz allgemein in Bereichen, in denen ein starker Zuwachs der Pflanzen unerwünscht ist.

Wichtig ist auch die Anwendung von Wachstumsregulatoren zur Hemmung des Längenwachstums von Getreide. Hierdurch wird die Gefahr des Umknickens ("Lagerns") der Pflanzen vor der Ernte verringert oder vollkommen beseitigt. Außerdem können Wachstumsregulatoren bei Getreide eine Halmverstärkung hervorrufen, die ebenfalls dem Lagern entgegenwirkt. Die Anwendung von Wachstumsregulatoren zur Halmverkürzung und Halmverstärkung erlaubt es, höhere Düngermengen auszubringen, um den Ertrag zu steigern, ohne daß die Gefahr besteht, daß das Getreide lagert.

Eine Hemmung des vegetativen Wachstums ermöglicht bei vielen Kulturpflanzen eine dichtere Anpflanzung, sodaß Mehrerträge bezogen auf die Bodenfläche erzielt werden können. Ein Vorteil der so erzielten kleineren Pflanzen ist auch, daß die Kultur leichter bearbeitet und beerntet werden kann.

Eine Hemmung des vegetativen Wachstums der Pflanzen kann auch dadurch zu Ertragsteigerungen führen, daß die Nährstoffe und Assimilate in stärkerem Maße der Blüten- und Fruchtbildung zugute kommen als den vegetativen Pflanzenteilen.

Mit Wachstumsregulatoren läßt sich häufig auch eine Förderung des vegetativen Wachstums erzielen. Dies ist von großem Nutzen, wenn die vegetativen Pflanzenteile geerntet werden. Eine Förderung des vegetativen Wachstums kann aber auch gleichzeitig zu einer Förderung des generativen Wachstums führen, dadurch daß mehr Assimilate gebildet werden, sodaß mehr oder größere Früchte entstehen.

Ertragssteigerungen können in manchen Fällen durch einen Eingriff in den pflanzlichen Stoffwechsel erreicht werden, ohne daß sich Änderungen des vegetativen Wachstums bemerkbar machen. Ferner kann mit Wachstumsregulatoren eine Veränderung der Zusammensetzung der Pflanzen erreicht werden, was wiederum zu einer Qualitätsverbesserung der Ernteprodukte führen kann. So ist es beispielsweise möglich, den Gehalt an Zucker in Zuckerrüben, Zuckerrohr, Ananas sowie in Zitrusfrüchten zu erhöhen oder den Proteingehalt in Soja oder Getreide zu steigern. Auch ist es beispielsweise möglich, den Abbau erwünschter Inhaltsstoffe, wie z.B. Zucker in Zuckerrüben oder Zuckerrohr, mit Wachstumsregulatoren vor oder nach der Ernte zu hemmen. Außerdem läßt sich die Produktion oder der Abfluß von sekundären Pflanzeninhaltsstoffen positiv beeinflussen. Als Beispiel sei die Stimulierung des Latexflusses bei Gummibäumen genannt.

Unter dem Einfluß von Wachstumsregulatoren kann es zur Ausbildung parthenokarper Früchte kommen. Ferner kann das Geschlecht der Blüten beeinflußt werden. Auch kann eine Sterilität des Pollens erzeugt werden, was bei der Züchtung und Herstellung von Hybridsaatgut eine große Bedeutung hat.

Durch den Einsatz von Wachstumsregulatoren läßt sich die Verzweigung der Pflanzen steuern. Einerseits kann durch Brechen der Apikaldominanz die Entwicklung von Seitentrieben gefördert werden, was besonders im Zierpflanzenbau auch in Verbindung mit einer Wuchshemmung sehr erwünscht sein kann. Andererseits ist es aber auch möglich, das Wachstum der Seitentriebe zu hemmen. Für diese Wirkung besteht z.B. großes Interesse im Tabakanbau oder bei der Anpflanzung von Tomaten.

Unter dem Einfluß von Wachstumsregulatoren kann der Blattbestand der Pflanzen so gesteuert werden, daß ein Entblättern der Pflanzen zu einem gewünschten Zeitpunkt erreicht wird. Eine derartige Entlaubung spielt bei der mechanischen Beerntung der Baumwolle eine große Rolle ist aber auch in anderen Kulturen wie z.B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Ebenso läßt sich mit Wachstumsregulatoren der Fruchtfall steuern. Einerseits kann ein vorzeitiger Fruchtfall verhindert werden. Andererseits kann aber auch der Fruchtfall oder sogar das Abfallen der Blüten bis zu einem gewünschten Maße gefördert werden ("Ausdünnung"), um die Alternanz zu brechen. Unter Alternanz versteht man die Eigenart einiger Obstarten, endogen bedingt von Jahr zu Jahr sehr unterschiedliche Erträge zu bringen. Schließlich ist es möglich, mit Wachstumsregulatoren zum Zeitpunkt der Ernte die zum Ablösen der Früchte erforderlichen Kräfte zu reduzieren, um eine mechanische Beerntung zu ermöglichen oder eine manuelle Beerntung zu erleichtern.

Mit Wachstumsregulatoren läßt sich ferner eine Beschleunigung oder auch Verzögerung der Reife des Erntegutes vor oder nach der Ernte erreichen. Dieses ist von besonderem Vorteil, weil sich dadurch eine optimale Anpassung an die Bedürfnisse des Marktes herbeiführen läßt. Weiterhin können Wachstumsregulatoren in manchen Fällen die Fruchtausfärbung verbessern. Darüberhinaus kann mit Wachstumsregulatoren auch eine zeitliche Konzentrierung der Reife erzielt werden. Damit werden die Voraussetzungen dafür geschaffen, daß z.B. bei Tabak,

Tomaten oder Kaffee eine vollständige mechanische oder manuelle Beerntung in einem Arbeitsgang vorgenommen werden kann.

Durch Anwendung von Wachstumsregulatoren kann ferner die Samen- oder Knospenruhe der Pflanzen beeinflußt werden, sodaß die Pflanzen, wie z.B. Ananas oder Zierpflanzen in Gärtnereien, zu einem Zeitpunkt keimen, austreiben oder blühen, an dem sie normalerweise hierzu keine Bereitschaft zeigen. Eine Verzögerung des Austriebes von Knospen oder der Keimung von Samen mit Hilfe von Wachstumsregulatoren kann in frostgefährdeten Gebieten erwünscht sein, um Schädigungen durch Spätfröste zu vermeiden.

Schließlich kann mit Wachstumsregulatoren eine Resistenz der Pflanzen gegen Frost, Trockenheit oder hohen Salzgehalt des Bodens induziert werden. Hierdurch wird die Kultivierung von Pflanzen in Gebieten möglich, die hierzu normalerweise ungeeignet sind.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch vermischen der Wirkstoffe mit Streckmitteln, also flüssigen Lösungsmitteln, unter Druck stehenden verflüssigten Gasen und / oder festen Trägerstoffen, gegebenenfalls unter Verwendung von oberflächenaktiven Mitteln, also Emulgiermitteln und / oder Dispergiermitteln und / oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier- und / oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycol-ether, Alkylsulfonate, Alkylsulfate, Arylsulfonate sowie

Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azol-Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen o,1 und 95 Gewichtsprozent Wirkstoff, vorzugsweise zwischen o,5 und 9o %.

Die erfindungsgemäßen Wirkstoffe können in den Formulierungen in Mischung mit anderen bekannten Wirkstoffen vorliegen, wie Fungizide, Insektizide, Akarizide und Herbizide, sowie in Mischungen mit Düngemitteln und anderen Wachstumsregulatoren.

Die Wirkstoffe können als solche, in Form ihrer Formulierungen oder der daraus bereiteten Anwendungsformen, wie gebrauchsfertige Lösungen, emulgierbare Konzentrate, Emulsionen Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Die Aufwandmengen können in einem größeren Bereich variiert werden. Im allgemeinen verwendet man pro Hektar Bodenfläche o,ol bis 5o kg, bevorzugt o,o5 bis lo kg an Wirkstoff.

Für die Anwendung gilt, daß die Anwendung der Wachstumsregulatoren in einem bevorzugten Zeitraum vorgenommen wird, dessen genaue Abgrenzung sich nach den klimatischen und vegetativen Gegebenheiten richtet.

Die Herstellung der erfindungsgemäßen 1-Amino-cyclopropancarbonsäure-Derivate der Formel (I) geht aus den nachfolgenden Beispielen hervor.

Herstellungsbeispiele

Beispiel 1

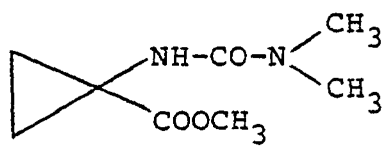

4,6 g (0,04 Mol) 1-Aminocyclopropancarbonsäuremethylester, 4,3 g Dimethylcarbamoylchlorid und 4,4 g Triethylamin werden in 50 ml Chloroform über Nacht unter Rückfluß erhitzt. Nach Abkühlung wäscht man die Reaktionsmischung zweimal mit 20 ml Wasser. Die organische Phase wird abgetrennt, über Natriumsulfat getrocknet und das Lösungsmittel abgezogen. Der Rückstand wird aus Methanol/Ether umkristallisiert. Man erhält 3,9 g (52 % der Theorie) an 1-(N-Dimethylaminocarbonyl-amino)-cyclopropan-carbonsäure-methylester in Form farbloser Kristalle vom Schmelzpunkt 115-117°C.

Beispiel 2

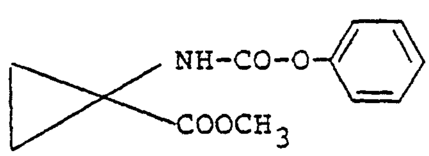

4,6 g (0,04 Mol) 1-Aminocyclopropancarbonsäuremethylester, 4,4 g Triethylamin und 9,4 g (0,06 Mol) Chlorameisensäurephenylester werden in 50 ml Toluol 3 Stunden unter Rückfluß erhitzt. Man kühlt auf Raumtemperatur ab, saugt ab und zieht das Lösungsmittel und überschüssige Chlorameisensäurephenylester ab. Der Rückstand wird aus Methanol/Ether umkristallisiert. Man erhält 6,6 g (70 % der Theorie) an N-Phenoxycarbonyl-1-aminocyclopropan-1-carbonsäuremethylester vom Schmelzpunkt 90-92°C.

Beispiel 3

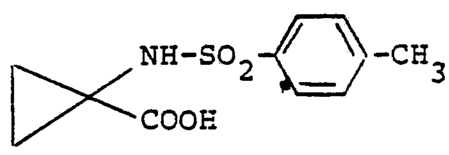

101 g (1 Mol) 1-Aminocyclopropancarbonsäure und 260 g (1,37 Mol) p-Toluolsulfonylchlorid werden in 870 ml 1-n-Natronlauge und 220 ml Wasser 3 Stunden bei Raumtemperatur gerührt. Während dieser Zeit hält man den pH-Wert durch Zugabe von 1-n-Natronlauge auf 9. Anschließend saugt man ab, stellt das Filtrat durch Zugabe von 1-N-Salzsäure auf pH 3, saugt ab, wäscht mit Wasser nach, trocknet und erhält 178 g (70 % der Theorie) an N-Tosyl-1-aminocyclopropancarbonsäure. Schmelzpunkt 200°C bis 203°C.

Beispiel 4

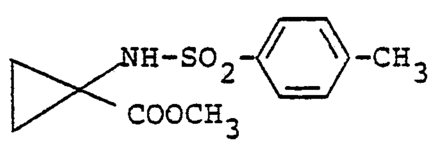

Nach der im Beispiel 3 angegebenen Methode wird auch der N-Toxyl-1-amino-cyclopropan-carbonsäure-methylester erhalten.

Beispiel 5

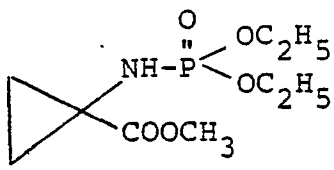

4,6 g (0,04 Mol) 1-Aminocyclopropancarbonsäuremethylester, 4,4 g Triethylamin und 6,9 g Chlorphosphonsäurediethylester werden in 50 ml Toluol 3 Stunden unter Rückfluß erhitzt. Man kühlt auf Raumtemperatur, saugt von ausgefallenen Salz ab und destilliert den Rückstand. Man erhält 6,0 g (60 % der Theorie) einer Verbindung der oben angegebenen Formel.

$K_{p\ 0,01} = 110°C$

Nach der im Beispiel 5 angegebenen Methode werden auch die in den folgenden Beispielen 6 bis 9 formelmäßig aufgeführten Stoffe der Formel (I).

Beispiel 6

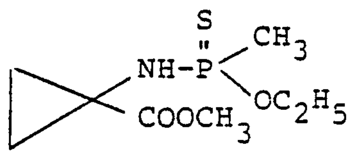

Beispiel 7

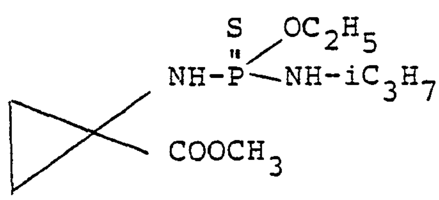

Beispiel 8

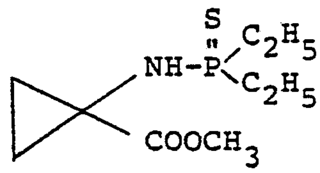

Beispiel 9

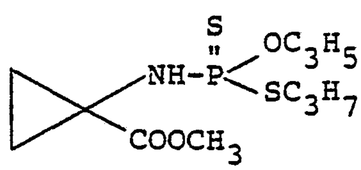

Beispiel 10

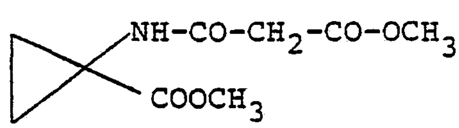

3,5 g (0,03 Mol) 1-Aminocyclopropancarbonsäuremethylester und 11,9 g (0,09 Mol) Malonsäuredimethylester werden 3 Stunden bei 100-110°C gerührt. Anschließend destilliert man den überschüssigen Malonester ab und kristallisiert den Rückstand aus Methanol/Ether um. Man erhält 4,1 g (63 % der Theorie) an einer Substanz der oben angegebenen Konstitution.

Schmelzpunkt: 68-70°C.

Beispiel 11

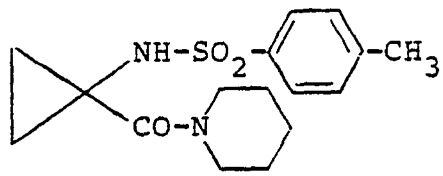

In 2,7 g (0,01 Mol) N-Tosyl-1-aminocyclopropancarbonsäurechlorid und 1,1 g Triethylamin in 20 ml hohem Toluol läßt man 1 g (0,012 Mol) Piperidin zutropfen. Man rührt über Nacht bei Raumtemperatur, filtriert vom Salz ab und engt das Filtrat ein. Der rückstand wird aus Methanol umkristallisiert. Man erhält 2,1 g (66 % der Theorie) an N-Tosyl-1-aminocyclopropancarbonsäurepiperidid vom Schmelzpunkt 195-197°C.

Beispiel 12

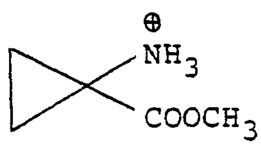

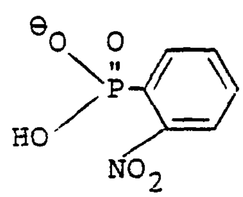

2,0 g (0,1 Mol) o-Nitrophenylphosphonsäure werden in 5 ml Methanol gelöst und mit 1,1 g 1-Aminocyclopropancarbonsäuremethylester versetzt. Man zieht das Lösungsmittel ab und erhält 3,2 g der Substanz der oben angegebenen Konstitution. Schmelzpunkt: 143-146°C

Beispiel IV-1

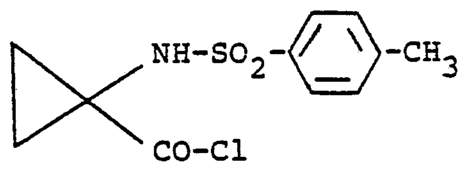

107 g (0,9 Mol) Thionylchlorid werden zu einer Mischung aus 114,8 g (0,45 Mol) 1-Tosylamino-cyclopropancarbonsäure und 1 l Toluol bei 20°C tropfenweise gegeben. Das Reaktionsgemisch wird 3 Stunden bei 80°C gerührt, dann wird das Lösungsmittel unter vermindertem Druck abdestilliert. Man erhält 115,7 g (94 % der Theorie) 1-Tosylamino-cyclopropancarbonsäurechlorid als öligen Rückstand.

Elementaranalyse:

| | | | | | |
|---|---|---|---|---|---|
| $C_{ber.}$ | 48,22 % | $H_{ber.}$ | 4,38 % | $Cl_{ber.}$ | 12,97 % |
| $C_{gef.}$ | 47,3 % | $H_{gef.}$ | 4,3 % | $C_{gef.}$ | 13,1 % |

Patentansprüche

1. 1-Amino-cyclopropancarbonsäure-Derivate der Formel

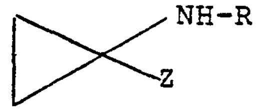

(I)

in welcher

Z für Cyano oder für den Rest $-CO-R^1$ steht, worin

$R^1$ für Hydroxy, Alkoxy, Alkenoxy, Alkinoxy. Aralkoxy, Aryloxy, oder für den Rest $-O^{\ominus}M^{\oplus}$ steht, wobei

$M^{\oplus}$ für ein Metallionenäquivalent oder für ein gegebenenfalls substituiertes Ammonium-, Sulfonium- oder Phosphonium-ion steht, und

$R^1$ weiterhin für den Rest 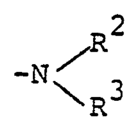 steht, worin

$R^2$ und $R^3$ unabhängig voneinander für Wasserstoff oder für gegebenenfalls substituierte Reste aus der Reihe Alkyl, Cycloalkyl, Alkenyl, Alkinyl, Aralkyl oder Aryl stehen oder

$R^2$ und $R^3$ zusammen mit dem N-Atom an das sie gebunden sind, für einen gegebenenfalls substituierten, gesättigten oder ungesättigten, gegebenenfalls

benzannellierten Mono- oder bicyclus, welcher gegebenenfalls 1 bis 3 weitere Stickstoffatome oder ein Sauerstoff- oder Schwefelatom als Heteroatom(e) enthält, stehen und

R für gegebenenfalls substituiertes Alkyl, gegebenenfalls substituiertes Aralkyl, einen gegebenenfalls substituierten heterocyclischen Rest oder den Rest $-CO-R^4$ steht, worin

$R^4$ für substituiertes Alkyl, gegebenenfalls substituiertes Aryl, einen gegebenenfalls substituierten Aminorest, für gegebenenfalls substituiertes Alkoxy oder für gegebenenfalls substituiertes Aryloxy steht, und

R weiterhin für den Rest $-S(O)_n-R^5$ steht, worin

n für 0, 1 oder 2 steht und

$R^5$ für gegebenenfalls substituiertes Alkyl, gegebenenfalls substituiertes Aryl oder für einen gegebenenfalls substituierten Aminorest steht und

R ferner für den Rest 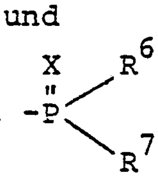 steht, worin

X für Sauerstoff oder Schwefel steht,

$R^6$ für gegebenenfalls substituiertes Alkyl, Aryl, Alkoxy oder Aryloxy steht und

$R^7$ für gegebenenfalls substituierte Reste aus der Reihe Alkoxy, Aryloxy, Alkylthio, Arylthio, Alkylamino, Arylamino, Alkyl oder Phenyl steht, und

R außerdem auch für Wasserstoff steht, mit der Maßgabe, daß die Verbindung der Formel (I) dann salzartig mit einer Sauerstoffsäure von Schwefel oder Phosphor, welche gegebenenfalls organische Reste enthält, verbunden ist.

2. Verfahren zur Herstellung von 1-Amino-cyclopropancarbonsäure-Derivaten der Formel (I) gemäß Anspruch 1, dadurch gekennzeichnet, daß man

a) 1-Amino-cyclopropancarbonsäure-Derivate der Formel

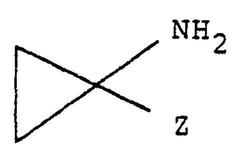

(II)

in welcher

Z die oben angegebene Bedeutung hat,

mit Verbindungen der Formel

$$Y-R^8 \qquad (III)$$

in welcher

$R^8$ die oben angegebene Bedeutung von R hat, jedoch nicht für Wasserstoff steht, und

Y für Halogen oder eine andere nucleofuge Gruppe steht,

gegebenenfalls in Gegenwart eines Säurebindemittels und gegebenenfalls in Gegenwart eines Verdünnungsmittels umsetzt, oder

b) 1-Amino-cyclopropancarbonsäurechloride der Formel

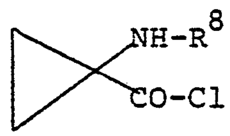

(IV)

in welcher

$R^8$ die oben angegebene Bedeutung von R hat, jedoch nicht für Wasserstoff steht,

mit Verbindungen der Formel

$$H-R^1 \qquad (V)$$

in welcher

$R^1$ die oben angegebene Bedeutung hat,

gegebenenfalls in Gegenwart eines Säurebindemittels und gegebenenfalls in Gegenwart eines Verdünnungsmittels umsetzt, oder

c) 1-Amino-cyclopropancarbonsäure-Derivate der Formel

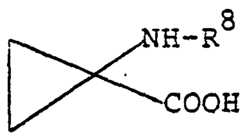

(VI)

in welcher

$R^8$ die oben angegebene Bedeutung von R hat, jedoch nicht für Wasserstoff steht,

mit Metallsalzen oder mit gegebenenfalls substituierten Ammonium-, Sulfonium- oder Phosphonium-Salzen gegebenenfalls in Gegenwart eines Säurebindemittels und gegebenenfalls in Gegenwart eines Verdünnungsmittels umsetzt, oder

d) 1-Amino-cyclopropancarbonsäure-Derivate der Formel

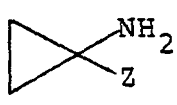

(II)

in welcher

Z die oben angegebene Bedeutung hat,

mit Sauerstoffsäuren von Schwefel oder Phosphor, welche gegebenenfalls organische Reste enthalten, gegebenenfalls in Gegenwart eines Verdünnungsmittels umsetzt.

3. Mittel zur Regulierung des Pflanzenwachstums, gekennzeichnet durch einen Gehalt an mindestens einem 1-Amino-cyclopropan-carbonsäure-Derivat der Formel (I) gemäß Anspruch 1.

4. Verfahren zur Regulierung des Pflanzenwachstums, dadurch gekennzeichnet, daß man 1-Amino-cyclopropan-carbonsäure-Derivate der Formel (I) gemäß Anspruch 1 auf die Pflanzen oder ihren Lebensraum ausbringt.

5. Verwendung von 1-Amino-cyclopropan-carbonsäure-Derivaten der Formel (I) gemäß Anspruch 1 zur Regulierung des Pflanzenwachstums.

6. Verfahren zur Herstellung von pflanzenwachstumsregulierenden Mitteln, dadurch gekennzeichnet, daß man 1-Amino-cyclopropancarbonsäure-Derivate der Formel (I) gemäß Anspruch 1 mit Streckmitteln und/oder oberflächenaktiven Stoffen vermischt.

7. 1-Amino-cyclopropancarbonsäurechloride der Formel

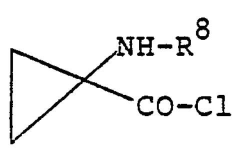

(IV)

in welcher

$R^8$ für gegebenenfalls substituiertes Alkyl, gegebenenfalls substituiertes Aralkyl, einen gegebenenfalls substituierten heterocyclischen Rest oder den Rest $-CO-R^4$ steht, worin

$R^4$ für substituiertes Alkyl, gegebenenfalls substituiertes Aryl, einen gegebenenfalls substituierten Aminorest, für gegebenenfalls substituiertes Alkoxy oder für gegebenenfalls substituiertes Aryloxy steht, und

$R^8$ weiterhin für den Rest $-S(O)_n-R^5$ steht, worin

n für 0, 1 oder 2 steht und

$R^5$ für gegebenenfalls substituiertes Alkyl, gegebenenfalls substituiertes Aryl oder für einen gegebenenfalls substituierten Aminorest steht, und

$R^8$ ferner für den Rest 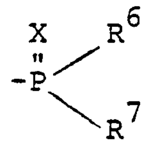 steht, worin

X für Sauerstoff oder Schwefel steht,

$R^6$ für gegebenenfalls substituiertes Alkyl, Aryl, Alkoxy oder Aryloxy steht und

$R^7$ für gegebenenfalls substituierte Reste aus der der Reihe Alkoxy, Aryloxy, Alkylthio, Arylthio, Alkylamino, Arylamino, Alkyl oder Phenyl steht.

8. Verfahren zur Herstellung von 1-Amino-cyclopropancarbonsäure-chloriden der Formel (IV) gemäß Anspruch 7, dadurch gekennzeichnet, daß man 1-Amino-cyclopropancarbonsäure-Derivate der Formel

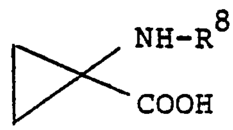

(VI)

in welcher

$R^8$ die oben angegebene Bedeutung hat,

mit einem Chlorierungsmittel gegebenenfalls in Gegenwart eines Verdünnungsmittels, sowie gegebenenfalls in Gegenwart eines Katalysators umsetzt.

Europäisches Patentamt

**EUROPÄISCHER RECHERCHENBERICHT**

0030287

Nummer der Anmeldung

EP 80107100.2

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| P | DE - A1 - 2 824 517 (BAYER) (06-12-1979)<br>+ Patentansprüche 1-6 +<br>& PT-A- 69 646 (01-05-1979)<br>& IL-A- 57 452 (30-09-1979)<br>-- | 1-6,8 |
| | DE - A1 - 2 558 078 (SCHERING)<br>+ Patentanspruch 1 +<br>-- | 1 |
| | US - A - 4 076 840 (PER ARVID EMIL CARLSSON)<br>+ Patentanspruch 1 +<br>-- | 1 |
| | US - A - 3 598 868 (DONALD J. CRAM)<br>+ Patentanspruch 1 +<br>---- | 1 |

KLASSIFIKATION DER ANMELDUNG (Int. Cl.³)

C 07 C 143/78
C 07 C 127/15
C 07 C 125/067
C 07 F 9/24
C 07 C 103/46
C 07 F 9/28
A 01 N 53/00

RECHERCHIERTE SACHGEBIETE (Int. Cl.³)

C 07 C 127/00
C 07 C 125/00
C 07 C 143/00
C 07 F
C 07 C 103/00
A 01 N 53/00

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| WIEN | 23-01-1981 | REIF |

EPA form 1503.1 06.78